# EUROPÄISCHE PATENTSCHRIFT

(11) **EP 1 424 097 B1**
(45) Veröffentlichungstag und Bekanntmachung des Hinweises auf die Patenterteilung: **21.06.2006**
(21) Anmeldenummer: 03027107.6
(22) Anmeldetag: 26.11.2003
(51) Int. Cl.: A61N 1/36, A61N 1/32, A61H 39/00

(54) **Reizstromstimulator zur Behandlung des Reizdarmsyndroms**
Stimulation current device for the treatment of irritable bowel syndrome
Appareil à courant de stimulation pour le traitement des irritations du côlon

(30) Priorität: 28.11.2002 DE 10255571
(43) Veröffentlichungstag der Anmeldung: 02.06.2004
(73) Patentinhaber: Schoo, Georg, 48356 Nordwalde (DE)
(72) Erfinder: Schoo, Georg, 48356 Nordwalde (DE)
(74) Vertreter: Habbel, Ludwig

(56) Entgegenhaltungen:
- EP-A- 0 211 146
- DE-A- 3 326 429
- DE-A- 3 813 838
- US-A- 4 676 246
- US-A1- 2002 058 972
- US-A1- 2002 077 688

## Beschreibung

Die Erfindung bezieht sich auf einen Reizstromstimulator zur Behandlung des Reizdarmsyndroms gemäß dem Oberbegriff des Patentanspruches 1.

Das Reizdarmsyndrom beschäftigt seit langem die Medizin. Hierbei handelt es sich um eine sogenannte funktionelle Erkrankung des Magen-Darm-Traktes.

Typisch für ein Reizdarmsyndrom ist, wenn sich die Symptome bei längerer Phase der Entspannung, z. B. im Urlaub, bessern und sich in Zeiten, in denen der Betroffene starkem Streß ausgesetzt ist, verschlimmern. Zu den Symptomen des Reizdarmsyndromes zählen Schmerz, Veränderungen im Stuhlgang, nämlich Verstopfung oder Durchfall sowie geblähter Bauch und auch Schleimabgaben.

Die Behandlung des Reizdarmsyndroms erfolgt über eine Arzneimitteltherapie, durch Umstellen der Ernährung und/oder durch Selbsthilfe, wobei zu der Selbsthilfe auch Therapieformen zählen, die die Psyche beeinflussen, d. h. Psychotherapie, Hypnose, autogenes Training, Atemübungen, funktionelle Entspannung sowie progressive Muskelrelaxsationen.

Um eine spezielle Bewegungsaktivität des Darmes zu erreichen, sind Dickdarmmassagen, Atemgymnastik und Bauchpressen bekannt.

Aus der DE 37 37 250 A1 ist ein Darmmassagegerät bekanntgeworden, bei welchem ein Unwuchtvibrator eingesetzt wird, der durch die Abstimmung der Geschwindigkeit und des Unwuchtgewichtes eine möglichst große Schwingweite erreicht. Hierdurch soll ein Schwingungseffekt erzielt werden, der angeblich aus der Vibrationsfördertechnik bekannt ist.

Bei vorgegebener und nicht variierbarer Schwingungsintensität soll ein für das Zielorgan spezifisch geformter Vibrationskopf durch mechanische Irritation der Darminnervierung die natürliche Darmperistaltik anregen, wobei sich zu einer Irritationswirkung der durch Selbstanwendung individuelle Druck auf den Darm in Richtung des natürlichen Transportweges des Darminhaltes wünschenswert addiert.

Das Gerät ist in Hantelform ausgebildet und weist einen am Körper anzuwendenden Vibrationskopf, der einen Schwingungsmotor enthält, und einen gegenüberliegenden Haltekopf auf, der als Batterielager zur manuellen Führung dient.

Ein solcher "Vibrator" hat sich aber zur Behandlung des Reizdarmsyndroms nicht bewährt.

Heute ist es bekannt, voraussagbar und gezielt sowie in Leistung und Zeit gesteuert, mit Hilfe von Reizstromstimmulatoren über Hautelektroden einen oder mehrere Nerven zur Auslösung von Aktionspotentialen damit zu einer Reaktion an seinem Erfolgsorgan zu zwingen. Die Reizauslösung, d. h. die Erzeugung eines nervalen Aktionspotentials durch Öffnen der Natriumlonenkanäle der Nervenmembrane erfolgt durch einen negativen Spannungsimpuls, da durch den erzeugten Stromfluß-Ladungsverschiebung eine Depolerisation des Ruhemembran-Potentials erzeugt wird.

Zum Einsatz kommen heute bei einer Muskelstimulation (d. h. bei einer Reizung mit dem Ziel, eine Kontraktion eines bestimmten Muskels zu erzeugen) monodirektionale (Gleichströme) und bidirektionale (Wechselströme) Impulsströme mit einem steilen Anstieg kurzer Impulszeiten (T kleiner als eine Millisekunde) und Frequenzen von ca. 1 - 60 hz. Im allgemeinen kommen heute zur Muskelstimulation, insbesondere im kosmetischen Bereich, vor allem kleine tragbare Stimulatoren vor, die mit bidirektional asymmetrischen Impulsen arbeiten.

Eine Vorrichtung zur Stimulation der Bauchmuskeln wird in der US-2002/0058972 A1 beschrieben. Hierbei werden wenigstens drei Elektroden auf einem Träger angeordnet und sollen die Unterleibsmuskeln stimulieren, wobei eine zentrale Elektrode vorgesehen ist, wobei zwei weitere Elektroden jeweils auf gegenüberliegenden Seiten dieser zentralen Elektrode angeordnet werden, so daß durch Aufbringen eines gepulsten Signals durch die zentrale Elektrode und die beiden Seitenelektroden, die Unterleibsmuskeln des zu Behandelnden beeinflußt werden.

Aus der DE 38 13 838 A1 ist ein Reizstromstimulator nach dem Oberbegriff von Anspruch 1 bekannt. Bei diesem elektromedizinischen Behandlungsgerät mit einer Stromquelle und einem Behandlungsteil sind die Einzelelektroden schachbrettartig angeordnet. Die Felder können sowohl rund als auch eckige Umfangslinien aufweisen. Durch eine solche Anordnung soll beispielsweise eine schneckenförmige Stromabfolge erzeugt werden, wodurch z. B. dem Darmverlauf folgend diese speziellen, bisher durch ständige Kreisbewegungen der Behandlungselektroden herbeigeführten darmanregenden Effekte erzielt werden.

Bei der Behandlung liegt ein Patient auf einer Basiselektrode auf, die aus mehreren Einzelelektroden bestehen kann. Gegenüberliegend ist die Behandlungselektrode angeordnet, wobei beide Elektrodenarten durch entsprechende Leitungen miteinander verbunden sind.

Der Erfindung liegt die Aufgabe zugrunde, einen vom Patienten zu tragenden Gürtel vorzuschlagen, der mit Elektroden ausgerüstet ist, die gezielt zum Einsatz zur Behandlung des Reizdarmsyndroms ausgebildet sind, wobei für diesen gezielten Einsatz eine bestimmte Form des Gerätes vorgeschlagen wird.

Diese der Erfindung zugrundeliegende Aufgabe wird durch die Lehre des Patentanspruches 1 gelöst.

Vorteilhafte Ausgestaltungen sind in den Unteransprüchen erläutert.

Somit wird ein Reizstromstimulator vorgeschlagen, bei welchem auf einer flexiblen Trägermatte Elektroden kreisförmig angeordnet sind, wobei die Trägermatte eine elektronische Steuerung trägt, die die einzelnen Elektroden aufeinanderfolgend für 4 bis 8 Sekunden in Tätigkeit setzt.

Die Anordnung der Elektroden ist so, daß diese der natürlichen Darmlage angepaßt angeordnet sind.

Es kann zusätzlich ein Heizelement vorgesehen sein, das in dem oberen Bereich des von den Elektroden besetzten Kreises vorgesehen ist und sich damit im wesentlichen im Bereich des Plexus Gastrici bzw. Plerus Solaris befindet. Dieses Heizelement kann als Wärmespeicher ausgebildet sein, beispielsweise ein steinartiger Wärmeträger, kann aber in gleicher Weise elektrisch betrieben werden oder durch ein Gel gebildet werden, das vor Inbetriebnahme der Gesamtanordnung aufgeheizt wird.

Um die Trägermatte und damit die Wirksamkeit der Elektroden an unterschiedliche Körpergrößen anpassen zu können, ist gemäß einem weiteren Merkmal der Erfindung vorgesehen, daß die Elektrodenanordnung in ihrer Weite einregelbar ist. Dies bezieht sich insbesondere auf die Breitenerstreckung der Elektroden voneinander, d. h. beispielsweise kann die Trägermatte in der Mitte geteilt sein, um nach Öffnung dieser Teilung, beispielsweise eines Reißverschlusses, auseinandergezogen zu werden, um somit die Größenanpassung vornehmen zu können.

Die eigentliche Trägermatte ist mit Halterungsmitteln ausgerüstet. Diese Halterungsmittel können aus Bänder bestehen oder auch in besonders vorteilhafter Weise aus einem Bügel, wie er bei Röntgenschürzen oder Haushaltschürzen bekannt ist, so daß ein einfaches Festklemmen des Stimulators auf dem Körper des Patienten möglich ist.

Die Impulszeiten der Elektroden sind einstellbar, ebenso wie die Impulsstärke und weiterhin ist eine Steuerung vorgesehen, die es ermöglicht, daß mehrere aufeinanderfolgende Elektrodendurchgänge einstellbar sind.

Bei einem bevorzugten Ausführungsbeispiel sind sieben Elektroden vorgesehen. Die Elektroden sind dabei unterschiedlich ausgebildet und weisen elliptische Form auf. Die einzelnen Elektroden werden nacheinander angesteuert, wobei im Ablauf die erste Elektrode elliptisch und relativ groß ausgebildet ist, die von einer kleineren elliptischen Elektrode gefolgt wird. Auf diese beiden Elektroden folgen zwei im wesentlichen nebeneinanderliegende kleinere Elektroden, die auf einer Kreissehne liegen. Im Ansteuerungsablauf folgt auf diese Elektroden eine größere Elektrode, deren Längsachse im wesentlichen quer zur Längsachse der beiden voraufgehenden Elektroden liegt. Um den Kreis zu schließen, folgen zwei kleinere Elektroden, wobei die letzte Elektrode, die im Enddarmbereich liegt, sehr klein ist.

Die erste Elektrode beginnt nicht mittig, sondern entsprechend der Darmlage versetzt schon auf dem aufsteigenden Bogen des gebildeten theoretischen Kreises.

Die aufeinanderfolgende Ansteuerung der Elektroden erfolgt derart, daß die beim Patienten unten etwa im Bereich des Blinddarmes liegende rechte Elektrode als erste in Betrieb genommen wird, dann nach kurzer Zeit und Stillsetzen dieser Elektrode die nächstfolgende aufsteigende Elektrode usw. bis das die auf den Patienten aufliegende linke untere Elektrode erreicht ist. Dieser Vorgang wird als ein Durchgang bezeichnet und nach einer Ruhepause kann ein zweiter und weitere Durchgänge erfolgen.

Jede Elektrode wird etwa 4 bis 8 Sekunden angesteuert und die Pause zwischen den einzelnen Durchgängen, wenn mehrere Durchgänge vorgesehen sind, beträgt etwa 5 - 20 Sekunden.

Durch die erfindungsgemäße Reizstimulation erfolgt eine Unterstützung der Darmtätigkeit, wobei als wichtiger Nebeneffekt gleichzeitig eine Stärkung der Bauchdecke erreicht wird, wobei diese Bauchdeckenstärkung sich auch wiederum positiv auf die Behandlung des Reizdarmsyndroms auswirkt, also im vorliegenden Fall nicht kosmetische Bedeutung hat.

Ein Ausführungsbeispiel der Erfindung wird nachfolgend anhand der Zeichnungen erläutert. Die Zeichnung zeigt in
- Fig. 1: eine Draufsicht auf die Trägermatte des Reizstromstimulators und in
- Fig. 2: die Unterseite der Matte, die mit dem Patienten in Kontakt kommt.

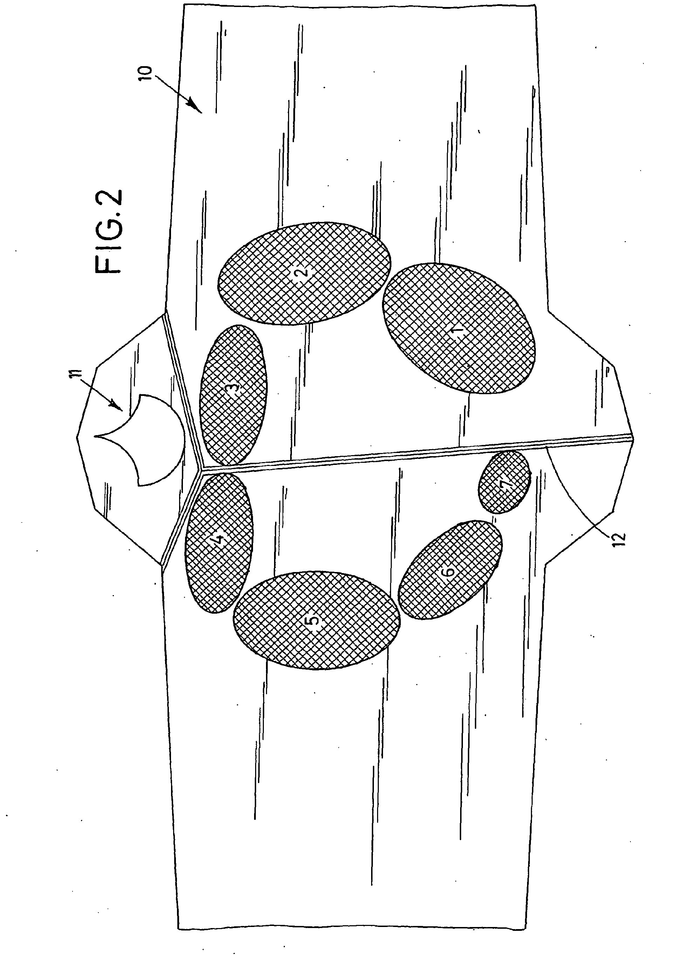

In den Zeichnungen ist mit 10 eine Trägermatte aus entsprechendem Werkstoff bezeichnet, in der Elektroden 1 bis 7 angeordnet sind. Diese Trägermatte 10 wird über in der Zeichnung nicht dargestellte Befestigungsmittel am menschlichen Körper so festgelegt, daß die unten liegende Seite, d. h. die Seite gemäß Fig. 2 mit dem menschlichen Körper in Kontakt kommt. Als Befestigungsmittel bieten sich Bänder oder vorzugsweise ein Bügel an, wie er bei Röntgenschürzen bekannt ist.

Um die Trägermatte 10 den unterschiedlichen menschlichen Größen anzupassen, ist über eine Verbindungseinrichtung 12, beispielsweise ein Reißverschluß oder ein Klettverschluß, die Matte 10 auseinanderziehbar, so daß damit bei dem dargestellten Ausführungsbeispiel die Breite der Matte 10 einstellbar ist.

In den Zeichnungen sind die verschiedenen Elektroden mit den Zahlen 1 bis 7 versehen. Diese Elektroden sind kreisförmig angeordnet und weisen selbst eine elliptische Form auf, wobei die Längsachse dieser Ellipse dem Verlauf des zu beeinflussenden Darmabschnittes angepaßt ist.

In den Zeichnungen sind die elektrischen Verbindungsleitungen und die elektronische Steuerung aus Übersichtlichkeitsgründen nicht dargestellt, wohl aber ein Schalter 14, mit dem die Intensität der Elektroden durch den Patienten einstellbar ist.

Zusätzlich liegt im oberen Bereich der Matte 10 eine Heizeinrichtung 11, die elektrisch betrieben werden kann oder aber auch als Gel ausgebildet sein kann, das durch eine Mikrowellenerwärmung erwärmt werden kann oder auch es kann ein Speicherkörper eingesetzt werden, beispielsweise ein Stein, der entsprechend erwärmt wird.

Die Elektroden werden in ihrer Reihenfolge 1 bis 7 aufeinanderfolgend betätigt, wobei nach einem Stromdurchgang durch die Elektroden 1 bis 7 eine kurze Pause eingeschaltet werden kann und dann erneut die Elektrodenabfolge wiederum betätigt wird.

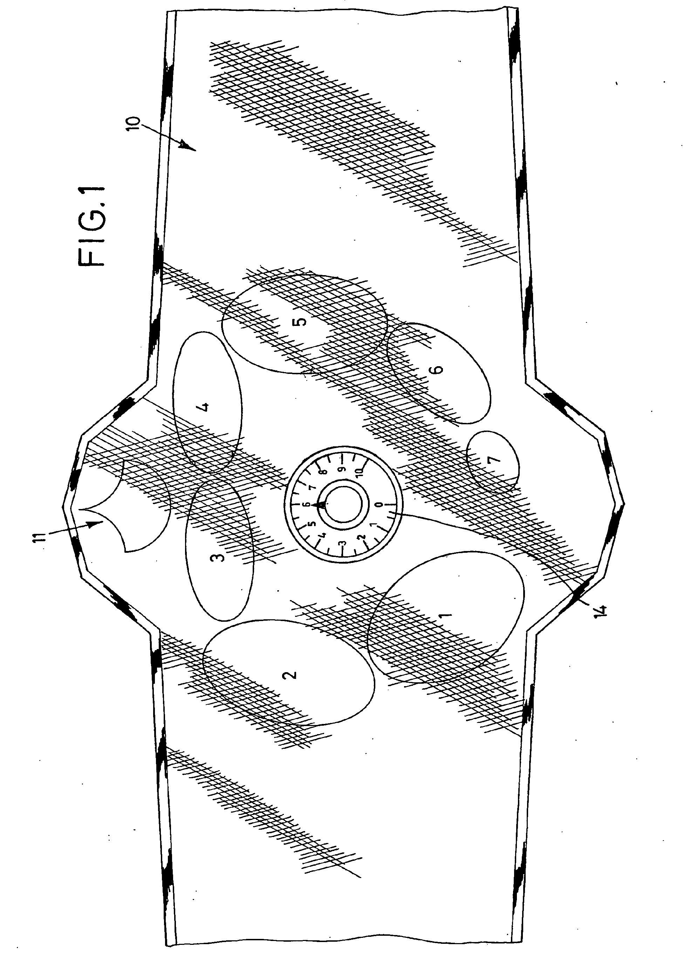

Die Elektrode 1 ist die größte, langgestreckte Elektrode, die bei der Draufsicht von außen gemäß Fig. 1 auf den Gürtel oder die Matte 10 von einer etwas kleineren langgestreckten Elektrode 2 gefolgt wird. Die Elektroden 3 und 4 sind wiederum kleiner als die Elektrode 2 und liegen auf der Sehne des theoretischen Kreises. An die Elektrode 4 schließt sich die Elektrode 5 an, die wiederum langgestreckt ausgebildet nach unten gerichtet ist. Die wiederum etwas kleinere Elektrode 6 schließt sich auf dem Kreisbogen an und wird von einer Elektrode 7 gefolgt, die im Bereich des Enddarmes liegt.

Beim Einsatz des erfindungsgemäßen Reizstromstimulators kommen die Elektroden 1 bis 7 gemäß Fig. 2 mit der Oberseite der Bauchdecke in Kontakt, vorzugsweise unter Zwischenschaltung eines Gels oder einer Flüssigkeit, um damit eine gute Stromübertragung sicherzustellen.

Jede einzelne Elektrode wird etwa 4 bis 8 Sekunden angesteuert, d. h. aufeinanderfolgend die Elektroden 1 bis 7 jeweils 4 bis 8 Sekunden. Nach einem Durchgang schließt sich eine Pause an, die etwa 5 bis 20 Sekunden betragen kann und dann erfolgt ein neuer Durchgang.

An dem in der Zeichnung dargestellten Steuergerät 14 ist sowohl die Intensität der Elektroden wie auch ggf. die Dauer der Wirksamkeit der Elektroden einstellbar und kann vom Patienten individuell eingestellt werden.

## Patentansprüche

1. Reizstromstimulator zur Behandlung des Reizdarmsyndroms mit auf einer flexiblen Trägermatte (10) der natürlichen Darmlage folgend angeordnete Elektroden (1 - 7) mit einer elektronischen Steuerung für eine Aktivierung der Elektroden in einer aufeinanderfolgenden Reihenfolge, **dadurch gekennzeichnet, daß** bei Verwendung jede Elektrode (1 - 7) 4 - 8 Sek. in Tätigkeit gesetzt wird und die Elektroden (1 - 7) elliptisch ausgebildet sind und auf der Trägermatte (10) mit ihrer langen Achse kreisförmig angeordnet sind, wobei bei Verwendung der obere Bereich des Kreises sich auf einem Köpper eines Patienten im Bereich des Plexus Gastrici und der untere Bereich des Kreises sich im Enddarmbereich befindet und die Ansteuerung der einzelnen Elektroden aufeinanderfolgend bei der im Bereich des Blinddarms liegenden, von vorne gesehen linken Elektrode (1) beginnt und an der im Enddarmbereich angeordneten Elektrode (7) endet.

2. Reizstromsimulator nach Anspruch 1, **dadurch gekennzeichnet, daß** aufeinanderfolgend, beginnend im Bereich des Blinddarmes, zwei große elliptische Elektroden (1, 2), anschließend zwei kleine elliptische Elektroden (3, 4) vorgesehen sind, auf die eine größere Elektrode (5) folgt, an die eine kleinere elliptische Elektrode (6) anschließt, wobei die abschließende Elektrode (7) im Enddarmbereich klein ausgebildet ist.

3. Reizstromstimulator nach Anspruch 1 oder 2, **gekennzeichnet durch** ein Heizelement (11) im elektrodenfreien Bereich des von den Elektroden (1 - 7) besetzten Kreises.

4. Reizstromstimulator nach einem oder mehreren der vorhergehenden Ansprüche, **dadurch gekennzeichnet, daß** die Trägermatte (19) mit Halterungsmittel ausgerüstet ist.

5. Reizstromstimulator nach Anspruch 4, **dadurch gekennzeichnet, daß** als Halterungsmittel ein Bügel eingesetzt ist.

6. Reizstromstimulator nach einem oder mehreren der vorhergehenden Ansprüche, **dadurch gekennzeichnet, daß** mehrere aufeinanderfolgende Elektrodendurchgänge in einem zeitlichen Abstand von 5 - 20 Sek. einstellbar sind.

7. Reizstromstimulator nach einem oder mehreren der vorhergehenden Ansprüche, **dadurch gekennzeichnet, daß** die Impulszeiten der Elektroden (1 - 7) einstellbar sind.

8. Reizstromstimulator nach einem oder mehreren der vorhergehenden Ansprüche, **dadurch gekennzeichnet, daß** die Impulsstärke der Elektroden (1 - 7) einstellbar ist.

## Claims

1. Stimulation current device for the treatment of irritable bowel syndrome, having electrodes (1 - 7) which are arranged on a flexible supporting mat (10) to follow the natural position of the intestines and which have a control means for activating the electrodes in a successive sequence, **characterised in that** each electrode (1 - 7) is put into action for 4 - 8 secs. in use and the electrodes (1 - 7) are of an elliptical form and are arranged on the supporting mat (10) with their long axes in a circle, with, in use, the upper region of the circle being situated on a patient's body in the region of the plexus gastrici and the lower region of the circle in the region of the rectum and the actuation of the individual electrodes in succession beginning with the electrode (1) which, seen from the front, is on the left and is situated in the region of the cecum and ending at the electrode (7) which is arranged in the region of the rectum.

2. Stimulation current device according to claim 1, **characterised in that** there are provided, in succession, beginning in the region of the cecum, two large elliptical electrodes (1, 2), and then two small elliptical electrodes (3, 4) which are followed by one larger electrode (5) which is succeeded by one smaller elliptical electrode (6), the final electrode (7) in the region of the rectum being of a small form.

3. Stimulation current device according to claim 1 or 2, **characterised by** a heating element (11) in the electrode-free region of the circle defined by the electrodes (1 - 7)

4. Stimulation current device according to one or more of the foregoing claims, **characterised in that** the supporting mat (19) is equipped with securing-on means.

5. Stimulation current device according to claim 4, **characterised in that** what is used as the securing-on means is a tying loop.

6. Stimulation current device according to one or more of the foregoing claims, **characterised in that** a plurality of successive electrode cycles can be set, at an interval of time of 5 - 20 secs.

7. Stimulation current device according to one or more of the foregoing claims, **characterised in that** the pulse times of the electrodes (1 - 7) can be set.

8. Stimulation current device according to one or more of the foregoing claims, **characterised in that** the pulse strength of the electrodes (1 - 7) can be set.

## Revendications

1. Appareil à courant de stimulation pour le traitement du syndrome du côlon irritable avec des électrodes (1 - 7) placées successivement sur un tapis de support souple (10) de la couche naturelle de l'intestin avec une commande électronique pour une activation des électrodes selon une séquence successive, **caractérisé en ce que**, lors de l'utilisation, chaque électrode (1 - 7) est mise en activité pendant 4 à 8 secondes et les électrodes (1 - 7) ont une forme elliptique et sont disposées en forme de cercle sur le tapis de support (10) avec leur axe long, moyennant quoi, lors de l'utilisation, la zone supérieure du cercle se trouve dans la zone du plexus gastrique sur le corps d'un patient et la zone inférieure du cercle se trouve dans la zone de l'appendice et l'excitation des électrodes individuelles les unes après les autres commence par l'électrode (1) se trouvant dans la zone du caecum, à gauche vue de l'avant, et se termine par l'électrode (7) disposée dans la zone de l'appendice.

2. Appareil à courant de stimulation selon la revendication 1, **caractérisé en ce qu'**il est prévu deux grandes électrodes elliptiques (1, 2) successives commençant dans la zone du caecum, puis de deux petites électrodes elliptiques (3, 4), qui sont suivies d'une plus grande électrode (5) à laquelle est raccordée une électrode elliptique plus petite (6), l'électrode finale (7) étant une petite électrode formée dans la zone de l'appendice.

3. Appareil à courant de stimulation selon la revendication 1 ou 2, **caractérisé par** un élément chauffant (11) dans la zone sans électrode du cercle occupé par les électrodes (1 - 7).

4. Appareil à courant de stimulation selon une ou plusieurs des revendications précédentes, **caractérisé en ce que** le tapis de support (19) est muni de moyens de retenue.

5. Appareil à courant de stimulation selon la revendication 4, **caractérisé en ce qu'**un étrier est utilisé comme moyen de retenue.

6. Appareil à courant de stimulation selon une ou plusieurs des revendications précédentes, **caractérisé en ce que** plusieurs passages d'électrodes successifs sont réglables dans un intervalle de temps de 5 à 20 secondes.

7. Appareil à courant de stimulation selon une ou plusieurs des revendications précédentes, **caractérisé en ce que** les temps d'impulsion des électrodes (1 - 7) sont réglables.

8. Appareil à courant de stimulation selon une ou plusieurs des revendications précédentes, **caractérisé en ce que** l'intensité d'impulsion des électrodes (1-7) est réglable.
